Europäisches Patentamt

**European Patent Office**

**Office européen des brevets**

(11) Publication number: **0 086 468**
A2

(12) **EUROPEAN PATENT APPLICATION**

(21) Application number: **83101313.1**

(22) Date of filing: **11.02.83**

(51) Int. Cl.³: **A 61 K 31/64**, A 61 K 47/00, A 61 K 9/48

(30) Priority: **15.02.82 GB 8204363**

(43) Date of publication of application: **24.08.83**
**Bulletin 83/34**

(84) Designated Contracting States: **AT BE CH DE FR GB IT LI LU NL SE**

(71) Applicant: **HOECHST U.K. LIMITED, Hoechst House Salisbury Road, Hounslow Middlesex TW4 6JH (GB)**

(72) Inventor: **Ganley, John Anthony, 2 Thames Drive Newport Pagnell, Milton Keynes Bucks. (GB)**
Inventor: **McTaggart, Celia Margaret, c/o Jesson 9 Dunbar Close Bletchley, Milton Keynes Bucks. (GB)**
Inventor: **Walker, Stephen Ernest, The Old Bakehouse Main Street, Maids Moreton Buckingham Bucks. (GB)**

(74) Representative: **Meyer-Dulheuer, Karl-Hermann, Dr. et al, HOECHST Aktiengesellschaft Zentrale Patentabteilung Postfach 80 03 20, D-6230 Frankfurt/Main 80 (DE)**

(54) **Oral anti-diabetic preparation.**

㊐ A pharmaceutical preparation which comprises a rigid shell containing an intrinsically short-acting oral anti-diabetic sulphonylurea derivative, in admixture with a pharmaceutically acceptable liquid carrier, the carrier comprising a hot melt having a melting point within the range of from 25°C to a temperature below that at which thermal damage is caused to the shell and a process for the manufacture thereof.

COMPLETE DOCUMENT

The present invention relates to pharmaceutical preparations comprising sulphonylurea derivatives.

Hypoglycaemic drugs containing the sulphonylurea grouping have been used for many years to lower blood sugar in maturity-onset diabetes mellitus. A number of compounds have been proposed, and some are widely used including tolbutamide, chlorpropamide and glibenclamide. Tolbutamide was for many years one of the leading products in the oral treatment of diabetes, but some patients with diabetes cannot be satisfactorily controlled with it. Chlorpropamide is also generally available and widely used. This compound is more potent than tolbutamide, but it has a relatively long plasma half-life of 24 to 72 hours. Although this reduces the number of daily doses necessary, the prolonged duration of effect associated with the long half-life increases the risk of hypoglycaemia, especially at night. Hypoglycaemia can be fatal, particularly in elderly patients, in whom the half-life may be even more prolonged.

A number of other sulphonylurea derivatives have a relatively long plasma half-life, but even in those having a relatively short half-life and therefore less risk of excessive hypoglycaemia, the time course of the blood sugar lowering action is still not optimum because there is a lag between the drug administration and the onset of its effect: the time of maximum blood sugar lowering is more than 2 hours after administration in many cases and may even be up to 6 hours.

Some attempts have been made to formulate faster-acting sulphonylurea derivatives in a quick-acting form to be taken before each meal, but none of the proposals has resulted in a preparation that has both a rapid onset of action and a short duration of drug effect.

- 3 -                    0086468

In British Patent 1 572 226 there is described a process wherein a mixture of a liquid carrier which is a molten mass with an active ingredient is dosed into a hard gelatin capsule. The dissolution of the drug and its biovailability depend on the nature of the liquid carrier.

According to British Patent 1 445 995 glibenclamide is dissolved or dispersed in a melt of a carrier for example polyethylene glycole and thereafter brought into a form suitable for oral administration. From the preparation the glibenclamide is immediately available for resorption. Since the preparations are prepared at elevated temperature an optimum of stability is not ensured.

It was therefore surprising, that glibenclamide can be used as active ingredient in the process generally discribed in the mentioned British Patent 1 572 226. The preparations do not show decomposition of glibenclamide as would be expected in knowledge of British Patent 1 445 995, and they show a rapid onset of action and a short duration of drug effect. It is known that the conventional glibenclamide containing preparation shows a long duration of drug effect and it is surprising that by the mentioned pharmaceutical technique the long lasting effect of glibenclamide formulations can be transformed to a short lasting one.

The present invention therefore, provides a pharmaceutical preparation containing in a rigid shell an intrinsically short-acting oral anti-diabetic sulphonylurea derivative in admixture with a pharmaceutically acceptable liquid carrier the carrier being a hot melt having a melting point within the range of from $25^{\circ}C$ to a temperature below that at which thermal damage is caused to the shell, generally having a melting point of from 30 to $90^{\circ}C$. The rigid shell is generally a hard gelatin capsule.

The term "an intrinsically short-acting oral anti-diabetic sulphonylurea derivative" is used herein to mean a sulphonylurea derivative having blood-sugar lowering effects when adminstered orally and having a plasma half-life, in conventional tablet form, of 10 hours or less. Examples of such agents are acetohexamide, tolbutamide, glibornuride, gliclazide, glibenclamide, glipizide and gliquidone ($\alpha$-form) and their physiologically acceptable salts. Of these, glibenclamide is particularly preferred.

The carrier for the sulphonylurea derivative is a substance which is brought by melting into liquid form for dosing into the rigid shell, and which solidifies sufficiently to lose its liquid properties after it has been dosed into the shell. The active ingredient and the carrier form a solid solution or solid dispersion of microfine crystals.

The carrier is a hot melt having a melting point generally within the range of from 30 to 90°C, preferably from 40 to 60°C. One or more substances may be used to produce a carrier having the desired melting point and flow properties; for example, the carrier may be a solid or semisolid substance itself having the desired hot melt properties, for example, a polyethylene glycol having a molecular weight in the range of from 400 to 20,000, for example from 1,000 to 6,000 (i.e. PEG 1000 to PEG 6000), or a mixture of two or more such polyethylene glycols. Alternatively, a liquid, solid or semi-solid substance may be used, to which one or more further substance(s) is or are added to give the desired properties. In such a case, the carrier base is generally one or more substances selected from polyethylene glycols each having a molecular weight in the range of from 400 to 20,000, and fixed oils and derivatives thereof, for example, arachis, rape, linseed and palm oils, and fractionated coconut oil.

Substances capable of modifying the melting point of a carrier base, sometimes called "hardeners" can be added. Examples for such substances are the following:

a) surfactants, for example, esters, for example, polyoxyethylene 40 stearate (Myrj 52), polyoxyethylene stearate (Myrj 51), sorbitan tristearate (Span 65), and polyoxyethylene (20) sorbitan mono-oleate (Tween 20); and ethers, for example, polyoxyethylene 20 cetyl ether (Bryj 38), polyoxyethylene 23 lauryl ether (Bryj 35), and alkyl phenol ethoxylates, for example Synperonic NP 20 (Myrj, Bryj, Span, Tween and Synperonic being Trade Marks);

b) sugars, for example, fructose, sorbitol and mannitol; and

c) organic amides, for example, urea.

Any two or more hardeners may be used. Particularly preferred is polyoxyethylene 40 stearate.

Polyethylene glycols (PEGs) are particularly useful as carriers, for example, the higher molecular weight PEGs, e.g. of molecular weight 1000 to 6000 may be used alone or in conjunction with a lower molecular weight PEG e.g. a PEG of molecular weight below 1000, for example PEG 6000 may be used with PEG 400. Polyoxyethylene 40 stearate is a preferred hardener for use with a PEG.

In the carrier one or more further substances may be incorporated, for example, selected from basic agents to assist the dissolution of the active ingredient, for example, sodium hydroxide, potassium hydroxide and triethanolamine; stabilising agent, for example, butylated hydroxytoluene, butylated hydroxyanisole, propyl gallate, E.D.T.A. disodium salt, and citric acid; and agents to adjust the ionic strength, for example, sodium chloride and potassium chloride.

Water may be incorporated to adjust the viscosity and/or hygroscopicity of the carrier.

The ratio between the total weight of the carrier and the weight of the active ingredient may be very wide and is, for example, in the range of from 20:1 to 200:1, preferably from 50:1 to 100:1.

The pharmaceutical preparation of the invention is generally produced by warming the carrier base and incorporating, in any order, any hardener(s) used, any other additives, and the active ingredient, and mixing until a homogeneous mix is obtained. The solution is preferably deaerated before it is filled into the rigid shells, generally hard gelatin capsules, on a suitable filling machine. The temperature for mixing and filling is in the range between $25^{\circ}C$ and below the temperature at which the rigid shell is damaged, and is generally within the range of from 30 to $90^{\circ}C$, preferably from 40 to $60^{\circ}C$. It will be appreciated that the temperature should be chosen so that the viscosity of the particular mix is suitable for filling.

A suitable filling machine may be prepared by modifying a conventional hard gelatin capsule filling machine by removing the filling head and replacing it by a device capable of dosing a predetermined amount of a liquid, for example, a refillable syringe, a peristaltic pump, a precision shot dispenser or a suitable liquid filler pump. It will be appreciated that it is necessary to provide heating means to ensure that the melt does not solidify or become unduly viscous before or during the dosing into the shell.

The preparations of the present invention have a good stability under temperate storage conditions. At a temperature of $20^{\circ}C$ they have a shelf life of at least 3 years. They do not need special storage conditions in countries having temperate climates.

The pharmaceutical preparations of the present invention have a number of advantages and supplement the assortment of the preparations at present on the market. One advantage is the consumer preference for capsules over tablets. Other advantages are: the uniformity in the dose of the active ingredient, the speed of onset and intensity of action of the sulphonylurea derivative, and the short duration of that activity.

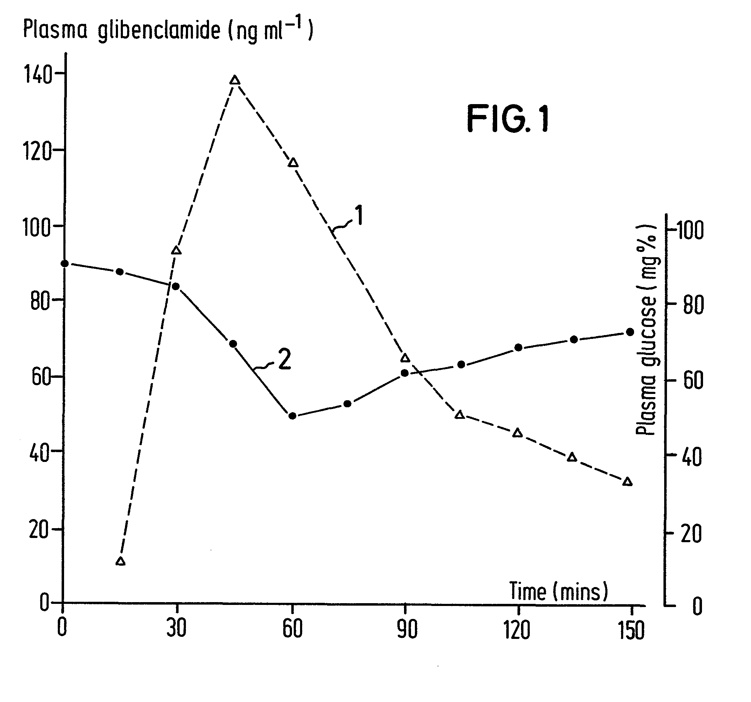

Figure 1 of the accompanying drawings shows the plasma glibenclamide levels (curve 1, mean of 14 subjects) and the plasma glucose levels (curve 2, mean of 6 fasting subjects). It is evident from Figure 1 that plasma glibenclamide levels rise to a peak 45 minutes after the adminstration of a preparation of the invention (preparation of Example 6), and that appreciable levels are present after only 30 minutes. The nadir of plasma glucose concentration in fasting subjects occurs 60 minutes after administration, ie. the delay between the peak plasma glibenclamide level and the plasma glucose nadir is only 15 minutes. This can be compared with published data on the time course of plasma glucose levels following adminstration of glibenclamide by an intravenous bolus injection, in the latter case, the peak glibenclamide plasma levels occur at the time of injection, but the nadir in plasma glucose levels is not seen until about 40 minutes thereafter (range 30 to 90 minutes). After oral adminstration of the preparation of the invention the same effect is observed as is the case after i.v. injection.

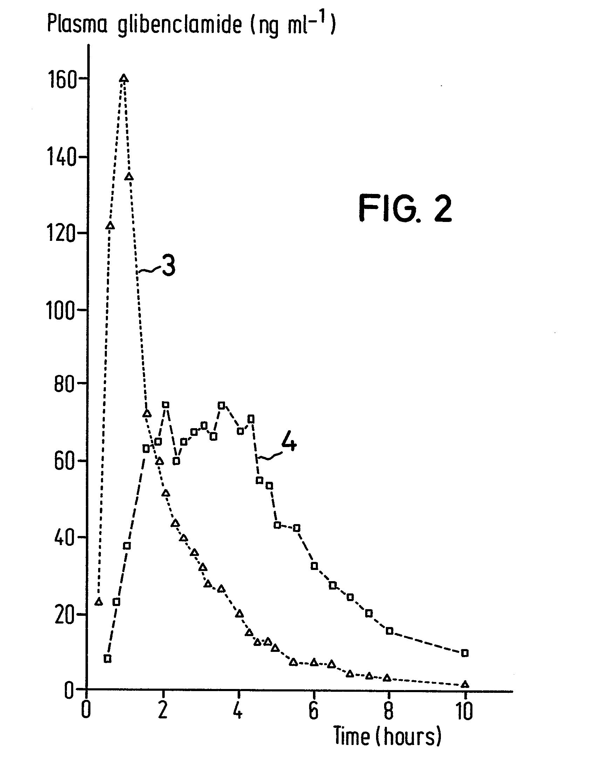

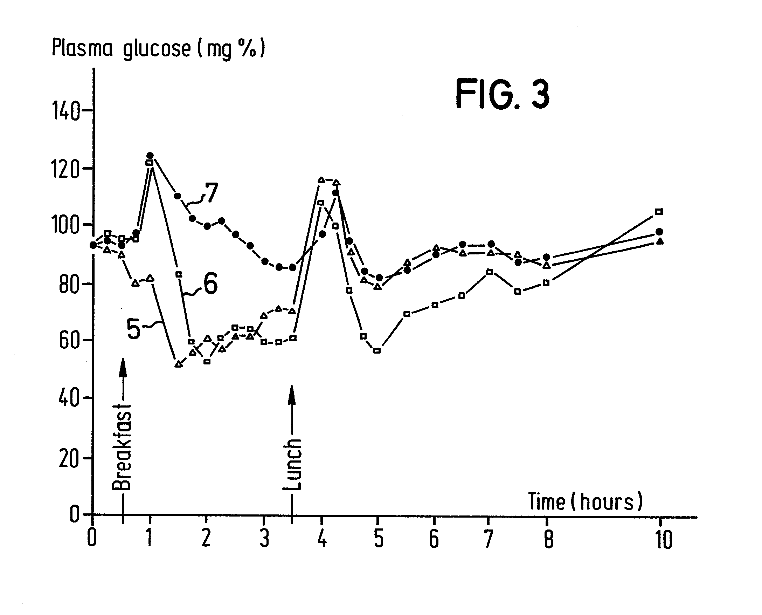

The second unexpected feature is the very short plasma half-life of the sulphonylurea derivative when present in a preparation of the present invention. This is illustrated in Figures 2 and 3 of the accompanying drawings.

Figure 2 shows the plasma glibenclamide levels (mean of six subjects) observed with the preparation of Example 6 (curve 3) and with a conventional glibenclamide tablet (curve 4). It can be seen that the plasma glibenclamide levels obtained with a preparation of the invention have reached a maximum (approximately twice that of the conventional tablet), and have already dropped to half of the maximum value before the plasma glibenclamide levels with the conventional preparation have reached their peak.

Figure 3 of the accompanying drawings shows the plasma glucose levels of 8 subjects treated with a conventional glibenclamide tablet (curve 6) and with the preparation of Example 6 (curve 5). As shown in Figure 3, the subjects were given breakfast 30 minutes after the adminstration of an active or placebo preparation (curve 7), and lunch 3 hours thereafter. From Figure 3, it can be seen that the preparation of the present invention starts reducing the blood sugar level 30 minutes after administration ie. when breakfast is taken, and completely eliminates the post-breakfast glucose peak, whereas with the conventional preparation, the blood sugar levels are still rising at this time, and only start to fall more than an hour after adminstration. The effect of the glibenclamide in the conventional preparation is still apparent at lunch time, serving to reduce the blood sugar level after lunch, whereas the blood sugar level observed after lunch in the case of the glibenclamide preparation of the present invention is, by this time, the same as with the placebo. The effect of the glibenclamide in the conventional preparation lasted for 8 hours, but only for 3.5 hours in the preparation of the present invention.

The preparations of the invention are preferable used in the treatment of diabetes mellitus.

The preparations of the present invention provide a blood sugar lowering effect that is both rapid in its onset and short lived. They are therefore particularly suitable for administration before each main meal, providing a "quasi-physiological" insulin release to cover meal-associated glucose peaks, but with a short duration of action which avoids inappropriate prolonged glucose lowering with its associated troublesome pre-prandial hypoglycaemia.

The preparations of the invention preferably comprise a unit dose of the sulphonylurea derivative suitable for lowering a meal-time blood glucose increase, for example, a suitable unit dose in the case of glibenclamide is preferably 0.5 to 6 mg. A unit dose is to be taken before each main meal, for example, up to 30 minutes before a meal, and normally three doses will be taken per day. It will be appreciated that using the preparations of the invention the treatment of diabetes mellitus can be much more flexible, for example, the timing of meals can be varied without the danger of hypoglycaemia.

The following Examples illustrate the invention. Instead of glibenclamide other sulphonylurea derivatives can be used.

EXAMPLE 1

| Ingredient | mg per capsule |
|---|---|
| Glibenclamide | 2.00 |
| PEG 400 | 147.60 |
| Myrj 52 | 138.40 |
| 1N NaOH solution | 10.44 |

Procedure

The PEG was warmed at $60^{o}$C until molten, then the glibenclamide was added and mixed thoroughly, followed by the sodium hydroxide solution and molten Myrj 52. The mixture was mixed at $60^{o}$C until homogeneous. The mixture was then deaerated before it was filled at $60^{o}$C into size 1 hard gelatin capsules on a hard gelatin capsule filling machine that had been modified by the replacement of the filling head by a precision shot dispenser or liquid filling pump.

EXAMPLE 2

| Ingredient | mg per capsule |
|---|---|
| Glibenclamide | 2.00 |
| PEG 400 | 24.80 |
| Bryj 38 | 23.20 |
| 1N NaOH solution | 10.44 |

Procedure

As described in Example 1, using Bryj 38 (polyoxyethylene 20 cetyl ether) instead of Myrj 52.

EXAMPLE 3

| Ingredient | mg per capsule |
|---|---|
| Glibenclamide | 1.00 |
| PEG 400 | 39.90 |
| Fructose | 34.60 |
| 1N NaOH solution | 5.22 |

Procedure

As described in Example 1, using fructose instead of Myrj 52.

EXAMPLE 4

| Ingredient | mg per capsule |
|---|---|
| Glibenclamide | 2.50 |
| PEG 6000 | 37.00 |
| 1N NaOH solution | 13.05 |

Procedure

The PEG was warmed at $60^{\circ}$C until molten. The glibenclamide was added and mixed thoroughly, followed by the sodium hydroxide solution. The filling was carried out as described in Example 1.

EXAMPLE 5

| Ingredient | mg per capsule |
|---|---|
| Glibenclamide | 0.50 |
| PEG 400 | 10.5 |
| Urea | 25.00 |
| Water | 4.0 |

Procedure

As described in Example 1, using urea instead of Myrj 52, and adding the water to the PEG 400.

EXAMPLE 6

| Ingredient | mg per capsule |
|---|---|
| Glibenclamide | 2.500 |
| PEG 400 | 184.500 |
| Myrj 52 | 173.000 |
| Distilled water | 23.333 |
| 1N NaOH solution | 13.044 |
| Propyl gallate | 0.370 |
| EDTA, disodium salt | 0.017 |

Procedure

As described in Example 1, adding the distilled water, the propyl gallate and the EDTA salt to the PEG 400.

EXAMPLE 7

| Ingredient | mg per capsule |
|---|---|
| Glibenclamide | 1.0 |
| Propylene glycol | 20.0 |
| PEG 6000 | 54.0 |
| Triethanolamine | 5.0 |

Procedure

As described in Example 1, adding the propylene glycol to the PEG 6000.

EXAMPLE 8

| Ingredient | mg per capsule |
|---|---|
| Glibenclamide | 2.0 |
| PEG 400 | 147.60 |
| Synperonic NP 20 | 138.40 |
| 1N NaOH solution | 10.44 |

Procedure

As described in Example 1, using Synperonic NP 20 (an alkyl phenol ethoxylate) instead of Myrj 52, and triethanolamine instead of sodium hydroxide.

EXAMPLE 9

| Ingredient | mg per capsule |
|---|---|
| Glibenclamide | 2.00 |
| PEG 4000 | 100.02 |
| PEG 400 | 12.61 |
| EDTA disodium salt | 0.01 |
| Potassium hydroxide | 0.56 |
| Distilled water | 4.80 |

Procedure

As described in Example 1, adding the EDTA and potassium hydroxide to the PEG melt.

EXAMPLE 10

| Ingredient | mg per capsule |
|---|---|
| Glibenclamide | 2.0 |
| PEG 400 | 200.0 |
| Colloidal silicon dioxide | 12.0 |
| Water | 4.8 |
| Sodium hydroxide | 0.4 |
| | 219.2 |

Method

Glibenclamide was dispersed in PEG 400 with aid of gentle heating. The solution of sodium hydroxide in water was added and mixed to give a clear solution. Then colloidal silicon dioxide was added and mixed until homogeneous. The mixture was filled into size 1 hard gelatin capsule while maintaining bulk under constant agitation to reduce viscosity. After filling viscosity increases by thixotropic conversion.

EXAMPLE 11

| Ingredient | mg per capsule |
|------------|---------------|
| Glibenclamide | 1.0 |
| PEG 400 | 100.0 |
| Thixcin R* | 7.5 |
| Distilled water | 2.4 |
| Sodium hydroxide | 0.2 |
| | 111.1 |

* An organic derivative of castor oil – N.L. Chemicals

Method

As described in Example 10 replacing colloidal silicon dioxide with Thixcin R.

CLAIMS:

1.    A pharmaceutical preparation which comprises a rigid shell containing an intrinsically short-acting oral anti-diabetic sulphonylurea derivative, in admixture with a pharmaceutically acceptable liquid carrier, the carrier comprising a hot melt having a melting point within the range of from 25$^{\circ}$C to a temperature below that at which thermal damage is caused to the shell.

2.    A pharmaceutical preparation as claimed in claim 1, wherein the sulphonylurea derivative is glibenclamide.

3.    A pharmaceutical preparation as claimed in claim 1, wherein the rigid shell is a hard gelatin capsule.

4.    A pharmaceutical preparation as claimed in claim 1, wherein the melting point of the carrier is within the range of from 30 to 90$^{\circ}$C, preferably within the range of from 40 to 60$^{\circ}$C.

5.    A pharmaceutical preparation as claimed in claim 1, wherein the carrier is one or more selected from polyethylene glycols having a molecular weight in the range of from 400 to 20,000, fixed oils and derivatives thereof.

6.    A pharmaceutical preparation as claimed in claim 1, wherein the carrier comprises a liquid, solid or semi-solid carrier base substance with which one or more further substance(s) is or are incorporated to bring the melting point within the defined range.

7.    A pharmaceutical preparation as claimed in claim 6, wherein the further substance(s) is or are selected from surfactants, sugars or organic acids.

0086468
HOE 82/S 012

8. A pharmaceutical preparation as claimed in claim 7, wherein the further substances are selected from polyoxyethylene 40 stearate, polyoxyethylene stearate, sorbitan tristearate, polyoxyethylene (20) sorbitan mono-oleate, polyoxyethylene 20 cetyl ether, polyoxyethylene 23 lauryl ether, an alkyl phenol ethoxylate, fructose, sorbitol, mannitol and urea.

9. A pharmaceutical preparation as claimed in claim 6, wherein the carrier comprises one or more polyethylene glycols having a molecular weight in the range of from 400 to 2000, and polyoxyethylene 40 stearate.

10. A pharmaceutical preparation as claimed in claim 1, which also comprises one or more further substances selected from basic substances, stabilising agents, agents to adjust the ionic strength, and water.

11. A pharmaceutical preparation as claimed in claim 1, wherein the ratio by weight total carrier: active ingredient is from 20 : 1 to 200 : 1, preferably from 50 : 1 to 100 : 1.

12. A pharmaceutical preparation as claimed in claim 1, which comprises from 0.5 to 6 mg of glibenclamide per unit dose.

13. A method of producing a pharmaceutical preparation as claimed in claim 1, which comprises warming the carrier or carrier base and incorporating in any order, any hardener(s) used, any other additive(s), and the active ingredient, mixing until a homogeneous mixture is obtained, and filling the mixture into a rigid shell.

Claims for Austria:

1. A method of producing a pharmaceutical preparation, which comprises warming a pharmaceutically acceptable liquid carrier or carrierbase, the carrier comprising a hot melt having a melting point within the range of from $25^{\circ}C$ to a temperature below that at which thermal damage is caused to a rigid shell and incorporating in any order, any hardener(s) used, any other additive(s), and as active ingredient an intrinsically short-acting oral anti-diabetic sulphonylurea derivative, mixing until a homogeneous mixture is obtained, and filling the mixture into a rigid shell.

2. A method as claimed in claim 1, wherein as the sulphonylurea derivative glibenclamide is used.

3. A method as claimed in claim 1, wherein a hard gelatin capsule is used as rigid shell.

4. A method as claimed in claim 1, wherein a carrier is used the melting point thereof is within the range of from 30 to $90^{\circ}C$, preferably within the range of from 40 to $60^{\circ}C$.

5. A method as claimed in claim 1, wherein the carrier used is one or more selected from polyethylene glycols having a molecular weight in the range of from 400 to 20,000, fixed oils and derivatives thereof.

6. A method as claimed in claim 1, wherein the further substance(s) used is or are selected from surfactants, sugars or organic acids.

7. A method as claimed in claim 1, wherein the further substances used are selected from polyoxyethylene 40 stearate, polyoxyethylene stearate, sorbitan tristearate, polyoxyethylene (20) sorbitan mono-oleate, polyoxyethylene 20 cetyl ether, polyoxyethylene 23 lauryl ether, an alkyl phenol ethoxylate, fructose, sorbitol, mannitol and urea.

8. A pharmaceutical preparation as claimed in claim 1, wherein the carrier used comprises one or more polyethylene glycols having a molecular weight in the range of from 400 to 2000, and polyoxyethylene 40 stearate.

9. A method as claimed in claim 1, wherein one or more further substances selected from basic substances, stabilising agents, agents to adjust the ionic strength, and water are used.

10. A method as claimed in claim 1, wherein the ratio by weight total carrier: active ingredient is from 20 : 1 to 200 : 1, preferably from 50 : 1 to 100 : 1.

11. A method as claimed in claim 1, wherein from 0.5 to 6 mg of glibenclamide per unit dose are used.

FIG.1

Plasma glibenclamide ($ng\,ml^{-1}$)

Plasma glucose ($mg\,\%$)

Time (mins)

0086468

2/3

Plasma glibenclamide (ng ml$^{-1}$)

FIG. 2

Time (hours)

FIG. 3